(19) Europäisches Patentamt
European Patent Office
Office européen des brevets

(11) **EP 4 035 731 B1**

(12) **EUROPEAN PATENT SPECIFICATION**

(45) Date of publication and mention
of the grant of the patent:
**08.01.2025   Bulletin 2025/02**

(21) Application number: **20867547.0**

(22) Date of filing: **24.09.2020**

(51) International Patent Classification (IPC):
*A61N 5/10* *(2006.01)*

(52) Cooperative Patent Classification (CPC):
**A61N 5/103;** A61N 2005/109

(86) International application number:
**PCT/CN2020/117285**

(87) International publication number:
**WO 2021/057828 (01.04.2021 Gazette 2021/13)**

(54) **IRRADIATION PARAMETER SELECTION APPARATUS AND USAGE METHOD THEREOF AND CONTROL SYSTEM COMPRISING SAID APPARATUS AND USAGE METHOD THEREOF**

BESTRAHLUNGSPARAMETERAUSWAHLVORRICHTUNG UND VERWENDUNGSVERFAHREN DAFÜR SOWIE STEUERUNGSSYSTEM MIT BESAGTER VORRICHTUNG UND VERWENDUNGSVERFAHREN DAFÜR

APPAREIL DE SÉLECTION DE PARAMÈTRES D'IRRADIATION ET SON PROCÉDÉ D'UTILISATION ET SYSTÈME DE COMMANDE COMPRENANT LEDIT APPAREIL ET SON PROCÉDÉ D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2019   CN 201910908146**
**25.09.2019   CN 201910908127**
**25.09.2019   CN 201910908121**

(43) Date of publication of application:
**03.08.2022   Bulletin 2022/31**

(73) Proprietor: **Neuboron Therapy System Ltd.**
**Xiamen, Fujian 211112 (CN)**

(72) Inventor: **LIU, Yuan-Hao**
**Nanjing, Jiangsu 211112 (CN)**

(74) Representative: **Gunzelmann, Rainer**
**Wuesthoff & Wuesthoff**
**Patentanwälte und Rechtsanwalt PartG mbB**
**Schweigerstraße 2**
**81541 München (DE)**

(56) References cited:
| | |
|---|---|
| WO-A1-2011/042819 | CN-A- 101 014 383 |
| CN-A- 104 353 195 | CN-A- 105 457 172 |
| CN-A- 106 853 272 | CN-A- 106 853 272 |
| CN-A- 106 853 272 | CN-A- 107 292 075 |
| CN-A- 107 666 940 | US-A1- 2018 277 278 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The disclosure relates to the technical field of radiotherapy, and in particular, to an irradiation parameter selection apparatus and a usage method thereof, a control system containing the apparatus and a usage method thereof.

BACKGROUND

With the development of atomic science, radiotherapy such as cobalt-60, linear accelerator, electron beam and the like has become one of the main means of cancer treatment. However, the conventional photonic therapy or electronic therapy is limited by physical conditions of the radiation itself, which can cause damage to a large number of normal tissues on the radiation beam pathway while killing tumor cells. In addition, due to differences in sensitivity of tumor cells to the radiations, the conventional radiation therapies tend to be less effective in the treatment to malignancies (e.g., the glioblastoma multiforme and the melanoma) which are relatively more radioresistant.

In order to reduce the radiation damage to normal tissues around the tumor, the target treatment concept in chemotherapy is applied to the radiotherapy. For the tumor cells with high radioresistance, radiation sources with a high relative biological effectiveness (RBE), such as proton therapy, heavy particle therapy, neutron capture therapy and the like, are also actively developed. The neutron capture therapy combines the above two concepts, such as boron neutron capture therapy (BNCT) which combines the specific aggregation of a boron-containing drug in tumor cells and the precise radiation beam regulation, to provide a better option than the conventional radiotherapies for the cancer treatment.

The boron neutron capture therapy utilizes a boron-containing ($^{10}$B) drug that has a high capture cross-section for thermal neutrons and utilizes $^{10}$B (n, $\alpha$) $^{7}$Li neutron capture and nuclear fission reaction to produce $^{4}$He and $^{7}$Li, the two heavy charged particles. The total range of the two particles is approximately equal to the size of a cell, so that the radiation damage to an organism can be limited to the cell level. When a boron-containing drug is selectively aggregated in tumor cells, combined with an appropriate neutron source, the purpose of locally killing the tumor cells can be achieved without causing too much damage to normal tissues.

In the present neutron capture therapy planning system, the irradiation geometrical angle is judged and defined manually according to experiences. Since the structure of a human body is quite complex and sensitivities of various tissues or organs to the radiation are greatly different, it is possible to ignore a better irradiation angle by human judgment alone, which leads to a great deterioration of the therapeutic effect. With the development of the technology, software began to be used to calculate evaluation values of several different irradiation angles and select an optimal irradiation point and angle accordingly. However, the optimal irradiation point and angle selected according to the calculation results of software are the theoretically optimal results which may be impossible applied in an actual operation. In order to achieve an optimal efficacy and satisfy the feasibility of the treatment plan, the selection of the irradiation point and the irradiation angle of the radiation beam needs to be further optimized.

Therefore, it is necessary to propose an irradiation parameter selection apparatus for selecting the optimal feasible irradiation point and irradiation angle.

In addition, prior to performing the neutron capture therapy, it is necessary to find the optimal feasible irradiation point and irradiation angle of the radiation beam, and then move the mounting table on which a patient is placed to an irradiation chamber for position adjustment until the position of the mounting table and the patient can be irradiated by the beam with the optimal feasible irradiation point and irradiation angle that are previously found. This process is cumbersome and time-consuming, which reduces the use efficiency of the neutron capture therapy equipment, and meanwhile, continuously adjusting the position of a patient for a long time makes the patient unbearable and makes the operator tired. In order to reduce the position adjustment time and improve the use efficiency of the equipment, the position adjustment process of the mounting table needs to be further optimized.

Therefore, it is necessary to propose a method for enabling quick adjustment of a mounting table in place.

US 2018/277278 AI, LIU YUAN-HAO [CN] ET AL, concerns an executable method for optimizing the irradiation angle, which can be used as a favorable reference, and in combination with doctor's experience, for finding out the best irradiation angle as far as possible. According to the method of D1 for evaluating the irradiation angle of the beam, it can be clearly recognized whether the performance of the beam irradiating at a certain position and at a certain angle is good or bad, thus providing strong data support for the doctor or the physicists to decide on the irradiation mode.

CN105457172 A, SHANGHAI UNITED IMAGING HEALTHCARE CO LTD, discloses a method for setting an irradiation angle in the radiation therapy. The method comprises the steps that an angle with maximum overall influences is selected from selectable angles, the selectable angles are angles which are manually input or automatically selected, and the overall influences refer to comprehensive influences brought by rays with unit intensity at the corresponding angle on tumors and organs at risk.

# EP 4 035 731 B1

## SUMMARY

The present invention is defined by the independent claims. Further aspects of the present invention are defined by the dependent claims. In order to overcome the drawbacks existed in the prior art, the first aspect of the disclosure provides an irradiation parameter selection apparatus capable of selecting an optimal feasible irradiation point and irradiation angle, in which irradiation parameters comprise irradiation points and irradiation angles, and the irradiation parameter selection apparatus comprises a sampling part for sampling multiple sets of irradiation points and irradiation angles; a calculation part for calculating an evaluation value corresponding to each set of irradiation point and irradiation angle; and a selection part for selecting one optimal feasible set of irradiation point and irradiation angle from all sampled irradiation points and irradiation angles according to the evaluation values calculated by the calculation part.

Further, the calculation part calculates a depth at which a neutron beam enters a patient and a type of an organ through which the neutron beam passes, and then determines whether a tumor is within a range of the maximum treatable depth corresponding to the set of the irradiation point and the irradiation angle according to track information of the neutron beam passing through a human body, if yes, calculates the evaluation value corresponding to the set of the irradiation point and the irradiation angle according to the track information in combination with data of the boron concentration in the organ, the radiation sensitivity factor of the organ, the characteristic information of the neutron beam and the like set by a user.

Further, the selection part removes unfeasible irradiation points and irradiation angles in an actual radiation process from all the sampled irradiation points and irradiation angles and selects the optimal feasible set of the irradiation point and irradiation angle.

Another aspect of the disclosure provides a usage method of the above said irradiation parameter selection apparatus, comprising the steps in which the sampling part reads an image of a patient, such as CT or MRI or PET-CT that has a clear anatomy of a human body, defines an outline of each organ, tissue and tumor one by one, provides settings of material type and density, and samplings an irradiation point and an irradiation angle of a neutron beam after defining the outline, material and density; the calculation part calculates a track in the organ through which the neutron beam passes, that is, calculates the type and thickness of the organ that the neutron beam passes through after entering the human body, determines whether the tumor is within the range of the maximum treatable depth after obtaining the track information of the neutron beam passing through the human body, if yes, calculates the evaluation value corresponding to the irradiation point and the irradiation angle according to the track information in combination with data of the boron concentration in the organ, the radiation sensitivity factor of organ, the characteristic information of the neutron beam and the like set by the user, if not, scores the worst evaluation value, and records the irradiation point, the irradiation angle and the corresponding evaluation value after the calculation of the evaluation value; and the selection part selects one optimal feasible set of the radiation parameters from all the sampled radiation parameters.

Further, the sampling of the irradiation points and the irradiation angles may be a forward sampling or a reverse sampling, in which a position of an irradiation point may be determined outside the human body in the forward irradiation point and the sampling may be made sequentially at a fixed angle interval or a fixed distance interval, or the sampling may be made randomly; and a position of an irradiation point may be determined within the range of a tumor in the reverse irradiation point such as at the centroid or the deepest point of the tumor, and a sampling of irradiation angles may be made by random sampling or at a predetermined angle interval; and a neutron beam angle may be set to a vector direction from the irradiation point to the centroid or the deepest point of the tumor.

Further, after sorting every set of irradiation point and irradiation angle, the selection part sequentially verifies whether each of the sets of irradiation points and irradiation angles is feasible from the best to the worst until the optimal feasible set of the irradiation point and irradiation angle is found.

Further, after the calculation of the evaluation values, the selection part firstly finds all of unfeasible irradiation points and irradiation angles, then removes the unfeasible irradiation points and irradiation angles, and finally selects the optimal set among the remaining irradiation points and irradiation angles.

Further, before the calculation of the evaluation values, the selection part removes all of unfeasible irradiation points and irradiation angles in advance, and selects the optimal set after the calculation is completed.

Further, the calculation part outputs the data of the irradiation points, the irradiation angles and the corresponding evaluation values in a form of 3D or 2D graph.

Further, the selecting process of the selection part may be performed entirely automatically by an associated device or may be partially manually performed.

The third aspect of the disclosure provides a control system for controlling a neutron capture therapy equipment comprising a mounting table for placing a patient, in which the control system is able to quick adjust the mounting table in place and comprises the irradiation parameter selection apparatus for selecting one optimal feasible set of an irradiation point and an irradiation angle; a conversion part for converting the parameters of the optimal feasible irradiation point and irradiation angle into coordinate parameters that the mounting table needs to be moved in place; and an adjustment part for adjusting the mounting table to a coordinate position obtained from the conversion part.

Further, the irradiation parameter selection apparatus comprises a sampling part, a calculation part and a selection part, in which the sampling part samplings multiple sets of irradiation points and irradiation angles; the calculation part calculates an evaluation value corresponding to each set of irradiation point and irradiation angle; and the selection part selects one optimal feasible set of irradiation point and irradiation angle from all sampled irradiation points and irradiation angles according to the evaluation values calculated by the calculation part.

Further, the conversion part converts the parameters of the optimal feasible radiation point and radiation angle into the coordinate parameters that the mounting table needs to be moved in place during the irradiation process according to CT/MRI/PET-CT information of the patient, positioning information, structure information of the mounting table and the like.

A fourth aspect of the present application provides a usage method of the control system, comprising the steps in which the irradiation parameter selection apparatus selects the optimal feasible irradiation point and irradiation angle; the conversion part converts the parameters of the optimal feasible irradiation point and irradiation angle into the coordinate parameters that the mounting table needs to be moved in place; and the adjustment part adjusts the mounting table to the coordinate position obtained from the conversion part.

Further, the irradiation parameter selection apparatus comprises a sampling part, a calculation part and a selection part, and the usage method of the irradiation parameter selection apparatus are as follows: first, the sampling part samplings multiple sets of irradiation points and irradiation angles; next, the calculation part calculates an evaluation value corresponding to each set of irradiation point and irradiation angle; and then the selection part selects the optimal feasible set of irradiation point and irradiation angle from all sampled irradiation points and irradiation angles according to the evaluation values calculated by the calculation part.

Further, the neutron capture therapy equipment irradiating a patient with a neutron beam to treat the patient, and the sampling part reads an image of the patient, such as CT or MRI or PET-CT that has a clear anatomy of a human body, defines an outline of each organ, tissue and tumor one by one, provides settings of material type and density, and samplings irradiation points and irradiation angles of the neutron beam after defining the outline, material and density.

Further, the calculation part calculates a track in the organ through which the neutron beam passes, that is, calculates the type and thickness of the organ that the neutron beam passes through after entering the body, determines whether the tumor is within the range of the maximum treatable depth after obtaining the track information of the neutron beam passing through the body, if yes, calculates the evaluation value corresponding to the irradiation point and the irradiation angle according to the track information in combination with the data of the boron concentration in the organ, the radiation sensitivity factor of organ, the characteristic information of the neutron beam and the like set by the user, if not, scoring the worst evaluation value, and records the irradiation point, the irradiation angle and the corresponding evaluation value after the calculation of the evaluation value

Further, the calculation part outputs the data of every set of the irradiation pointand the irradiation angle and the corresponding evaluation value in a form of 3D or 2D graph.

Further, after sorting every set of irradiation point and irradiation angle, the selection part sequentially verifies whether each of the sets of irradiation points and irradiation angles is feasible from the best to the worst until the optimal feasible set of the irradiation point and irradiation angle is found.

Further, the selection part first finds all of unfeasible radiation points and radiation angles, then removes the unfeasible radiation points and radiation angles, and last selects the optimal set of the radiation point and radiation angle among the remaining irradiation points and irradiation angles.

The fifth aspect of the disclosure provides a neutron capture therapy equipment capable of performing a judgment of the quality of a radiation point and a radiation angle, which comprises a neutron beam generating assembly, an irradiation chamber for irradiating a neutron beam to an irradiated object, a management chamber for performing irradiation control, a mounting table for placing a patient and a control system for controlling and managing a treatment process, in which the control system comprises an irradiation parameter selection apparatus for selecting an optimal radiation point and radiation angle, and the radiation parameter selection apparatus comprises a sampling part for sampling multiple sets of radiation points and radiation angles and a calculation part for calculating an evaluation value corresponding to each set of radiation point and radiation angle and outputting a report.

Further, the calculation part calculates a depth at which a neutron beam enters a patient and a type of an organ through which the neutron beam passes, and then determines whether a tumor is within a range of the maximum treatable depth corresponding to the set of the irradiation point and the irradiation angle according to track information of the neutron beam passing through a human body, if yes, calculates the evaluation value corresponding to the set of the irradiation point and the irradiation angle according to the track information in combination with data of the boron concentration in the organ, the radiation sensitivity factor of the organ, the characteristic information of the neutron beam and the like set by a user, if not, scores the worst evaluation value.

Further, the calculation part outputs the data of the irradiation points, the irradiation angles and the corresponding evaluation values in a form of 3D or 2D graph.

Further, corresponding to a certain irradiation point, a certain irradiation angle and a certain irradiation track, the weighting factor (W(i)) of the organ i is calculated with Equation 1:

$$W(i) = I(i) \times S(i) \times C(i) \qquad \text{(Equation 1)}$$

in which I(i), S(i) and C(i) are the neutron intensity, the radiation sensitivity factor of the organ i and the boron concentration in the organ i, respectively.

Further, I(i) is calculated with Equation 2 which integrates a depth intensity or a dose curve of a simulated body based on the beam used:

$$I(i) = \int_{x_0}^{x} i(x)dx \qquad \text{(Equation 2)}$$

in which i(x) is the depth intensity or the dose curve function of the beam for the treatment in an approximate body, and $x_0$-x is the depth range in the beam track of the organ i.

Further, an evaluation factor is calculated with Equation 3:

$$Q(x, y, z, \phi, \theta) = \sum_{i} W(i) \qquad \text{(Equation 3)}$$

in which Q(x, y, z, $\varphi$, $\theta$) as the evaluation factor is equal to the sum of the weighting factors of every organ in the organ-track.

Further, a ratio of the evaluation factor to the tumor evaluation factor (QR(x, y, z, $\varphi$, $\theta$)) is calculated with Equation 4:

$$QR(x, y, z, \phi, \theta) = \frac{\sum_{i} W(i)}{W(tumor)} \qquad \text{(Equation 4)}$$

in which W(tumor) is the weighting factor of the tumor.

The sixth aspect of the disclosure provides a usage method of the irradiation parameter selection apparatus, comprising the steps in which the sampling part reads an image of a patient, such as CT or MRI or PET-CT that has a clear anatomy of a human body, defines an outline of each organ, tissue and tumor one by one, provides settings of material type and density, and samplings an irradiation point and an irradiation angle of a neutron beam after defining the outline, material and density; the calculation part calculates a track in the organ through which the neutron beam passes, that is, calculates the type and thickness of the organ that the neutron beam passes through after entering the body, determines whether the tumor is within the range of the maximum treatable depth after obtaining the track information of the neutron beam passing through the body, if yes, calculates the evaluation value corresponding to the irradiation point and the irradiation angle according to the track information in combination with data of the boron concentration in the organ, the radiation sensitivity factor of organ, the characteristic information of the neutron beam and the like set by the user, if not, scores the worst evaluation value, and records the irradiation point, the irradiation angle and the corresponding evaluation value after the calculation of the evaluation value.

Further, the sampling of the irradiation points and the irradiation angles may be a forward sampling or a reverse sampling, in which a position of an irradiation point may be determined outside the body in the forward sampling and a sampling may be made sequentially at a fixed angle interval or a fixed distance interval, or the sampling may be made randomly; and a position of an irradiation point may be determined within the range of a tumor in the reverse sampling, in which the irradiation point may be at the centroid or the deepest point of the tumor, and a sampling of irradiation angles may be made by random sampling or at a predetermined angle interval; and a neutron beam angle may be set to a vector direction from the irradiation point to the centroid or the deepest point of the tumor.

Further, the calculation part outputs data of the irradiation points, the irradiation angles and the corresponding evaluation values in a form of 3D or 2D graph.

BRIEF DESCRIPTION OF THE DRAWINGS

FIG. 1 is a schematic diagram of a boron neutron capture reaction.

FIG. 2 is the neutron capture nuclear reaction equation of $^{10}$B (n, $\alpha$) $^7$Li.

FIG. 3 is a schematic diagram of the neutron capture therapy equipment in an example of the disclosure.

FIG. 4 is a schematic diagram of the control system in an example of the disclosure.

FIG. 5 is a logical block diagram of the calculation of an evaluation value of irradiation parameters of the neutron beam in an example of the disclosure.

FIG. 6 is a schematic diagram of the organ track during the neutron beam irradiation in an example of the disclosure.

DETAILED DESCRIPTION

Examples of the disclosure will now be described in further details with reference to the accompanying drawings in order to enable those skilled in the art to carry out the same with reference to the specification.

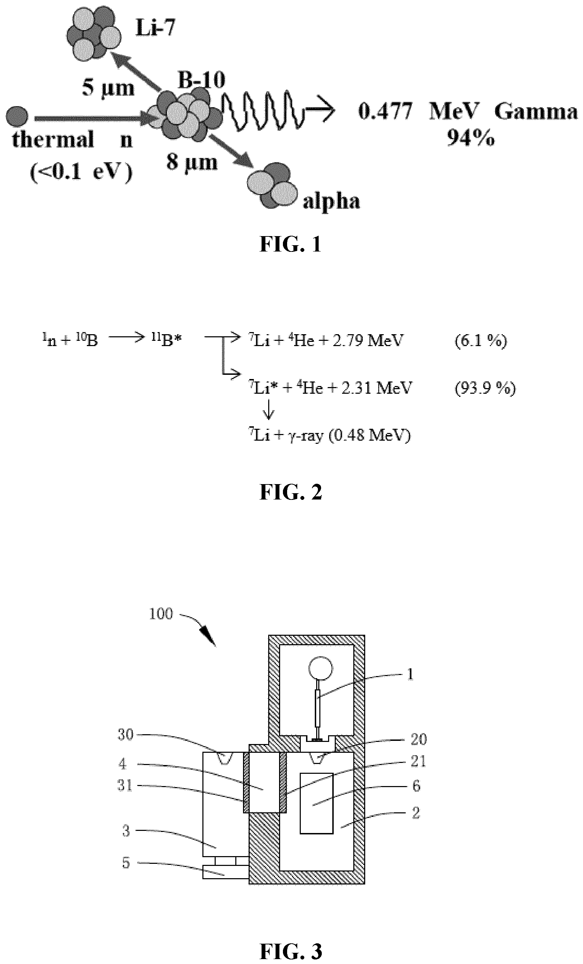

The neutron capture therapy has been increasingly used as an effective method for cancer treatment in recent years. The boron neutron capture therapy is the most common method, in which the neutrons for boron neutron capture therapy may be supplied by a nuclear reactor or an accelerator. The boron neutron capture therapy (BNCT) utilizes a boron-containing ($^{10}$B) drug that has a high capture cross-section for thermal neutrons and utilizes $^{10}$B (n, $\alpha$) $^7$Li neutron capture and nuclear fission reaction to produce two heavy charged particles of $^4$He and $^7$Li. Referring to FIG. 1 and FIG. 2, they show the schematic diagram of a boron neutron capture reaction and the $^{10}$B (n, $\alpha$) $^7$Li neutron capture and nuclear fission reaction equation, respectively. The two heavy charged particles have an average energy of about 2.33 MeV and the characteristics of a high linear transfer (LET) and a short range. The linear energy and the range of the $\alpha$ particle are 150 keV/$\mu$m and 8 $\mu$m, respectively, and the linear energy and the range of the $^7$Li heavy charged particle are 175 keV/$\mu$m and 5 $\mu$m, respectively. The total range of the two particles is approximately equal to the size of a cell, so that the radiation damage to an organism can be limited to a cell level. When a boron-containing drug is selectively aggregated in tumor cells, combined with an appropriate neutron source, the purpose of locally killing the tumor cells can be achieved without causing too much damage to normal tissues.

Whatever the neutron source for boron neutron capture therapy is from a nuclear reactor or from a nuclear reaction of charged particles with a target, a mixed radiation field is generated, that is, the radiation beam contains neutrons and photons from low energy to high energy. For boron neutron capture therapy for a tumor in a deep position of a body, the greater the amount of radiation other than epithermal neutrons, the greater the proportion of non-selective dose deposition to normal tissues. Therefore, the radiation that would cause unnecessary dose deposition should be minimized. In order to better understand the dose distribution of neutrons in the human body, in addition to air beam quality factors, a human head tissue prosthesis is used in the examples of the disclosure for dose distribution calculations, and the prosthetic beam quality factor is used as a design reference for the neutron beam.

The International Atomic Energy Agency (IAEA) provides five recommendations of air beam quality factors for neutron sources used in a clinical boron neutron capture therapy. The five recommendations can be used to compare the advantages and disadvantages of different neutron sources and as a reference for selecting neutron generation method and designing radiation beam shaping assembly. The five recommendations are as follows:

Epithermal neutron flux > 1 $\times$ 10$^9$ n/cm$^2$s;

Fast neutron contamination < 2 $\times$ 10$^{-13}$ Gy-cm$^2$/n;

Photon contamination < 2 $\times$ 10$^{-13}$ Gy-cm$^2$/n;

Thermal to epithermal neutron flux ratio < 0.05; and

Neutron current to flux ratio > 0.7.

Note: the epithermal neutron energy range is between 0.5eV and 40keV, the thermal neutron energy range is less than 0.5eV and the fast neutron energy range is greater than 40keV

1. Epithermal Neutron Flux :

The neutron flux and the boron-containing drug concentration in the tumor together determine the clinical therapy time. If the concentration of the boron-containing drug in the tumor is high enough, the requirement for the epithermal neutron flux can be reduced. Conversely, if the concentration of the boron-containing drug in the tumor is low, the requirement for the epithermal neutron flux should be high to deliver a sufficient dose to the tumor. The requirement for epithermal neutron flux suggested by the IAEA is that the number of epithermal neutrons per second per square centimeter is greater than $10^9$, such that the therapy time can be generally controlled within one hour for various current boron-containing drugs. The short therapy time is not only beneficial to patient positioning and comfort, but also can effectively utilize the limited residence time of boron-containing drugs in tumors.

2. Fast Neutron Contamination:

Since fast neutrons can cause an unnecessary normal tissue dose, they are considered to be contamination and the dose has a positive correlation with the neutron energy. Therefore, the amount of the fast neutrons should be minimized in a neutron beam design. The fast neutron contamination is defined as the fast neutron dose associated with a unit of the epithermal neutron flux, and the IAEA suggests that the fast neutron contamination should be less than $2 \times 10^{-13}$ Gy-$cm^2$/n.

3. Photon Contamination ($\gamma$ ray contamination):

$\gamma$ rays are strong penetrating radiation and can non-selectively cause dose deposition in all tissues along the neutron beam pathway. Therefore, reducing the amount of $\gamma$ rays is also a necessary requirement for the beam design. $\gamma$ ray contamination is defined as the $\gamma$ ray dose associated with a unit of epithermal neutron flux. The IAEA suggests that the $\gamma$ ray contamination should be less than $2 \times 10^{-13}$ Gy-$cm^2$/n.

4. Thermal Neutron To Epithermal Neutron Flux Ratio:

Since the thermal neutron decays fast and has a poor penetration capacity, most energy of the thermal neutron is deposited in the skin tissue after entering the human body. In addition to epidermal tumors such as melanoma, for which the thermal neutron may be used as a neutron source for the boron neutron capture therapy, with respect to the tumors located in a deep position of a body, such as a brain tumor, the thermal neutron amount should be reduced. The IAEA suggests that the ratio of the thermal neutron flux to the epithermal neutron flux should be less than 0.05.

5. Neutron Current To Flux Ratio:

The neutron current to flux ratio represents the directionality of a beam. The higher the ratio is, the better the forward direction of the beam is. The high forward directionality of the neutrons can reduce the dose in the surrounding normal tissues caused by neutron divergence. The IAEA suggests that the neutron current to flux ratio should be greater than 0.7.

The dosage distribution within tissues may be derived from a prosthesis, and the prosthesis beam quality factor may be derived from the dose-depth curves of a normal tissue and a tumor. The following three parameters can be used to compare the efficacy of different neutron beam therapies.

1. Effective Treatment Depth:

A tumor dose is equal to the depth of the maximum dose of normal tissues. The tumor cells located at a deeper position receive a dose less than the maximum dose of normal tissues, i.e. the advantage of boron neutron capture is lost. This parameter represents the penetration capacity of the beam, and the greater the effective treatment depth (in a unit of cm) is, the deeper the tumor can be treated.

2. Effective Treatment Depth Dose Rate:

Effective treatment depth dose rate, i.e., the tumor dose rate of the effective treatment depth, is equal to the maximum dose rate of the normal tissue. Since the total dose received by the normal tissue is a factor that affects the total dose given to the tumor, so that the parameters affect the length of the treatment time. The greater the effective treatment depth dose rate is, the shorter the irradiation time required to administrate a certain dose to the tumor is, with a unit of Gy/mA-min.

3. Effective Treatment Dose Ratio:

From the surface of the brain to the effective treatment depth, the ratio of the average dose received by the tumor to that received by normal tissues is referred to as the effective treatment dose ratio. The calculation of the average dose can be obtained by integrating the dose-depth curve. The greater the effective treatment dose ratio is, the better the efficacy of the beam is.

In order that designs of the beam shaping assembly have a standard for comparison, in addition to five air beam quality factors recommended by the IAEA and the three parameters mentioned above, the following parameters for evaluating the beam dose qualities are also used in the examples of the disclosure:

1. Irradiation time $\leq$30min (proton current used by the accelerator is 10mA);

2. 30.0RBE-Gy Treatable Depth $\geq$7cm;

3. Maximum tumor dose $\geq$60.0RBE-Gy;

4. Maximum normal brain tissue dose $\leq$12.5RBE-Gy; and

5. Maximum skin dose $\leq$11.0RBE-Gy.

Note: RBE is the relative biological effectiveness. Since the biological effectiveness caused by photons and neutrons are different, an equivalent dose may be obtained by multiplying the above respective dose terms with the relative biological effectiveness of different tissues.

As shown in FIG. 3, the neutron capture therapy equipment 100 for the neutron capture therapy has a neutron beam generating assembly 1, an irradiation chamber 2 for irradiating a neutron beam to an irradiated object, for example a patient, a preparation chamber 3 for performing preparation before the irradiation, a communication chamber 4 for communicating the irradiation chamber 2 with the preparation chamber 3, a management chamber 5 for performing irradiation control, a positioning device (not shown) for positioning the patient, a mounting table 6 for placing a patient to be moved in the preparation chamber 3 and the irradiation chamber 2, and a control system 7 for controlling and managing a treatment process.

The neutron beam generating assembly 1 is configured to generate a neutron beam outside the irradiation chamber 2 and to irradiate the neutron beam to a patient positioned in the irradiation chamber 2, in which a collimator 20 is provided. The preparation chamber 3 is a room for performing the preparation required before irradiating the neutron beam to the patient. The preparation chamber 3 is provided with an analog collimator 30. The preparation comprises fixing the patient on the mounting table 6, positioning the tumor of the patient, making three-dimensional positioning marks and the like. The management chamber 5 is a room for managing and controlling the overall treatment processes performed by the boron neutron capture therapy equipment 100. For example, a manager confirms the state of the preparation in the preparation chamber 3 from the management chamber 5 by naked eyes, and operates the control system 7 to control the start and stop of irradiation of the neutron beam and the position adjustment of the mounting table 6 to carry the patient to perform rotation, horizontal movement, and lifting movement. The control system 7 is only a general term. It may be a set of general control system, that is, the start and stop of the irradiation of the neutron beam, the position adjustment of the mounting table 6 and the like are controlled by one set of control system; or it may be several sets of control systems, that is, the start and stop of the irradiation of the neutron beam, the position adjustment of the mounting table 6 and the like are respectively controlled by several sets of control systems.

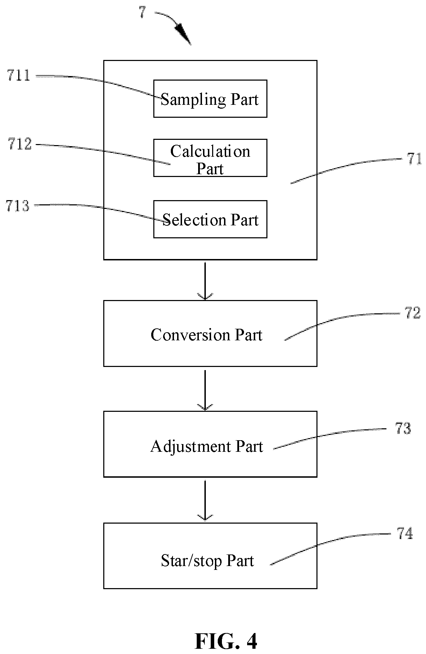

As shown in FIG. 4, before performing the boron neutron capture therapy, it is necessary for the manager to determine the angle from which the neutron beam irradiated the patient can kill the tumor cells to the maximum extent and reduce the damage of the radiation to the surrounding normal tissues as much as possible, and adjust the mounting table 6 on which a patient is placed to the corresponding position after determining the optimal feasible irradiation point and irradiation angle. Specifically, the control system 7 comprises an irradiation parameter selection apparatus 71 for selecting the optimal feasible irradiation point and irradiation angle, a conversion part 72 for converting the optimal feasible irradiation point and irradiation angle into coordinate parameters of the mounting table 6, an adjustment part 73 for adjusting the mounting table 6 to the coordinate position obtained from the conversion part 72, and a start-stop part 74 for controlling the start and stop of irradiation of the neutron beam.

Further referring to FIG. 4, each set of irradiation parameters includes an irradiation point and an irradiation angle of the neutron beam, and the irradiation parameter selection apparatus 71 includes a sampling part 711, a calculation part 712, and a selection part 713. First, the sampling part 711 samplings multiple sets of irradiation points and irradiation angles, and then the calculation part 712 calculates an evaluation value corresponding to each set of irradiation points and irradiation angles, and then the selection part 713 selects one optimal feasible set of irradiation parameters from all the sampled irradiation points and irradiation angles based on the evaluation value calculated by the calculation part 712. Specifically, the selection part 713 removes irradiation parameters that are not feasible in the actual treatment process and selects the optimal feasible set of irradiation parameters. The sampling part 711 may sampling the irradiation point and the irradiation angle randomly or regularly. The evaluation value calculation calculates the organ track of the neutron beam passing through the patient. That is, the calculation part 712 calculates the depth at which the neutron beam enters the human body and the type of the organ through which the neutron beam passes, and then determines whether the tumor is within the maximum treatable depth range corresponding to the set of irradiation parameters based on the track information of the neutron beam passing through the human body. If yes, the evaluation value corresponding to the set of irradiation point and the irradiation angle is calculated based on the data such as the boron concentration in the organ, the radiation sensitivity factor of the organ, the characteristic information of the neutron beam and the like set by the user. If not, the irradiation point and the irradiation angle are scored a particular evaluation value, and sampling and calculation of an irradiation point and an irradiation angle of the neutron beam are repeated. After the evaluation values corresponding to the irradiation points and the irradiation angles are calculated, the quality of each set of irradiation point and the irradiation angle can be apparently sorted according to the evaluation values. Since the position of collimator 20 is fixed and a device such as a positioning device may be further provided in the irradiation chamber 2, some certain positions of the patient may not be applicable and some certain movement positions of the mounting table may be interfered. In addition, certain parts of the patient, such as an organ, e.g. an eye, cannot be irradiated. Therefore certain irradiation points and irradiation angles cannot be used. In an actual treatment process, it is necessary to remove these irradiation points and irradiation angles which cannot be used by the selection part 713.

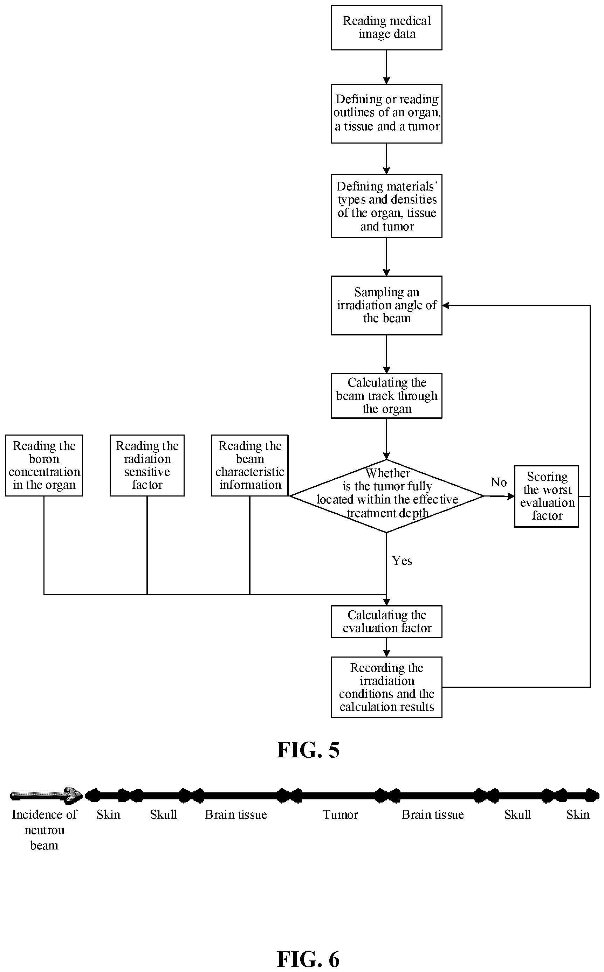

By referring to FIGS. 5 and 6, a method of using the irradiation parameter selection apparatus 71 will be described in detail. The method comprises the following steps. The sampling part 711 reads an image of a patient, such as CT or MRI or PET-CT that has a clear anatomy of a human body, defines an outline of each organ, tissue and tumor one by one, provides settings of material type and density, and samplings an irradiation point and an irradiation angle of a neutron beam after defining the outline, material and density. The sampling of the irradiation points and the irradiation angles may be a forward sampling or a reverse sampling, in which the position of an irradiation point may be determined outside the body in the forward sampling and a sampling may be made sequentially at a fixed angle interval or a fixed distance interval, or the sampling may be made randomly; a neutron beam angle is set to a vector direction from the irradiation point to the centroid or the deepest point of the tumor; and a position of an irradiation point may be determined within the range of a tumor in the reverse sampling in which the irradiaiton point is at the centroid or the deepest point of the tumor, and the sampling of irradiation angles may be made by random sampling or at a predetermined angle interval. After determining the irradiation point and the irradiation angle of the neutron beam, the calculation part 712 calculates a track in the organ through which the neutron beam passes, that is, calculates the type and thickness of the organ that the neutron beam passes through after entering the body, determines whether the tumor is within the range of the maximum treatable depth after obtaining the track information of the neutron beam passing through the body, if yes, calculates the evaluation value corresponding to the irradiation point and the irradiation angle according to the track information in combination with the data of the boron concentration in the organ, the radiation sensitivity factor of organ, the characteristic information of the neutron beam and the like set by the user, if not, scores a particular evaluation value, repeats the sampling of an irradiation point and an irradiation angle of the neutron beam, and records the irradiation point, the irradiation angle and the corresponding evaluation value after the calculation of an evaluation value. Repeating the sampling and calculation to a certain number, and outputting a report. The selection part selects one optimal feasible set of the radiation parameters from all the sampled radiation parameters. The calculation part 712 may output the data of the irradiation points, the irradiation angles and the corresponding evaluation values in a form of 3D or 2D graph. In this case, a doctor or a physician may more readily determine the quality of irradiation points and irradiation angles.

Preferably, after sorting every set of irradiation point and irradiation angle, the selection part 713 sequentially verifies whether each of the sets of irradiation points and irradiation angles is feasible from the best to the worst until the optimal feasible set of the irradiation point and irradiation angle is found. Of course, the selection part 713 may first find all of unfeasible radiation points and radiation angles after the calculation of the evaluation values, then remove the unfeasible radiation points and radiation angles, and finally select the optimal set of the radiation point and radiation angle among the remaining irradiation points and irradiation angles. The selection part 713 may also remove all of unfeasible irradiation points and irradiation angles before the calculation of the evaluation values, and select the optimal set of irradiation point and irradiation angle after the calculation is completed.

The selecting process may be performed entirely automatically by an associated device, or may be partially manually performed, or may be performed entirely manually, that is, the selection part 713 is not provided. For example, the unfeasible irradiation points and irradiation angles may be listed by an experienced doctor, or may be determined by simulation with associated devices. Sorting the evaluation values and operating the selection of the optimal irradiation point and irradiation angle after removing the unfeasible irradiation points and irradiation angles can also be determined by an experienced doctor or by associated devices. After obtaining the optimal feasible irradiation point and irradiation angle, the conversion part 72 converts the parameters of the optimal feasible irradiation point and irradiation angle into the coordinate parameters that the mounting table 6 needs to be moved in place during the irradiation in combination with the CT/MRI/PET-CT information of the patient, the position information, the structure information of mounting table 6 and the like. Then the adjustment part 73 adjusts the mounting table 6 to a predetermined position based on the coordinate information obtained from the conversion part 72. After the adjustment part 73 adjusts mounting table 6 to a predetermined position, the positioning device further confirms whether the irradiation point and the irradiation angle of the neutron beam with respect to the patient's tumor are the same as the pre-selected optimal feasible irradiation point and irradiation angle. If not, manually adjusts the patient position or the mounting table 6 position to ensure that the neutron beam irradiates the patient's tumor at the optimal feasible irradiation point and irradiation angle, or drives the adjustment part 73 to adjust the position of the mounting table 6 to ensure that the neutron beam irradiates the patient's tumor at the optimal feasible irradiation point and with the optimal feasible irradiation angle.

To prevent radiation in the irradiation chamber 2 from scattering outside the irradiation chamber 2, a first shielding door 21 is provided between the irradiation chamber 2 and the communication chamber 4, and a second shielding door 31 is provided between the communication chamber 4 and the preparation chamber 3. In other embodiments, a shielding wall with a labyrinth can replace the first shielding door and the second shielding door, and the shape of the labyrinth including, but not limited to a "Z" shape, a "bow" shape and a " EL" shape.

The specific example in which the evaluation value is calculated by the calculation part 712 will be described in details. Of course, the calculation part 712 is not limited to this example, and other methods and equations may be used to calculate the evaluation value. The evaluation value is calculated on the basis of the neutron beam characteristics, the organ radiation sensitivity factor and the boron concentration in the organ, the weighting factor ($W(i)$) of the organ i is calculated with Equation 1, in which $I(i)$, $S(i)$ and $C(i)$ are the neutron intensity, the radiation sensitivity factor of the organ i and the boron concentration of the organ i, respectively.

$$W(i) = I(i) \times S(i) \times C(i) \quad \text{(Equation 1)}$$

In Equation 1, $I(i)$ is obtained by integrating the depth intensity or dose curve of the neutron beam in the simulated human body, as shown in Equation 2, in which $i(x)$ is the depth intensity or the dose curve function of the beam for the treatment in an approximate body, and $x_0$-x is the depth range of the organ i in the beam track.

$$I(i) = \int_{x_0}^{x} i(x)dx \quad \text{(Equation 2)}$$

By the above-mentioned calculation, the evaluation value corresponding to the neutron beam can be obtained by sequentially calculating the weighting factors of every organ in the organ track and summing them up, as shown in Equation 3. In this calculation, the weighting factor of a tumor should not be included in the calculation.

$$Q(x, y, z, \phi, \theta) = \sum_{i} W(i) \quad \text{(Equation 3)}$$

According to the above-mentioned evaluation value, it is possible to more apparently judge the degree of harm to normal tissues during a treatment. In addition to evaluating the irradiation positions and angles using the evaluation value, an evaluation ratio factor which is defined as the ratio of the evaluation value to the tumor weighting factor, may also be used for the evaluation, as shown in Equation 4, with which the expected efficacy of the irradiation positions and angles can be sufficiently revealed.

$$QR(x, y, z, \phi, \theta) = \frac{\sum_{i} W(i)}{W(tumor)} \quad \text{(Equation 4)}$$

The above examples involve the steps of: "reading a patient image, such as CT/MRI/PET-CT or the like having a clear anatomy of a human body, defining the outline of every organ, tissue and tumor one by one, and providing settings of materials' types and densities". Details can be found for example in a patent application No. 201510790248.7 submitted before China National Intellectual Property Administration on November 17, 2015 with the title of "METHOD OF GEOMETRICAL MODEL ESTABLISHMENT BASED ON MEDICAL IMAGING DATA".

As is well known to those skilled in the art, some of the simple transformations in the above equations 1 to 4 are still within the scope of the disclosure. For example, $I(i)$, $S(i)$ and $C(i)$ may be transformed by multiplication into addition; $I(i)$, $S(i)$ and $C(i)$ may be multiplied by the power n, respectively, and n may be an integer multiple of 1 or other multiples, depending on requirements; and $i(x)$ may be an average number between $x_0-x$ or an intermediate number multiplied by $(x_0-x)$, or any calculation method that can achieve the result of the intensity integration calculation.

While the foregoing has described illustrative specific examples of the disclosure in order to facilitate an understanding of the disclosure by those skilled in the art. It is to be understood that the disclosure is not limited by the scopes of the specific examples. The invention is defined in the following claims. Other embodiments, examples, methods etc. are not a part of the invention.

**Claims**

1.   An irradiation parameter selection apparatus (71) for a neutron beam, wherein irradiation parameters comprise irradiation points and irradiation angles, wherein, the irradiation parameter selection apparatus (71) comprises:

a sampling part (711) for sampling multiple sets of irradiation points and irradiation angles, wherein a sampling of the irradiation points and the irradiation angles is a forward sampling, wherein a position of an irradiation point is determined outside the human body and the sampling is made sequentially at a fixed angle interval or a fixed distance interval, or the sampling is made randomly; or a reverse sampling, wherein a position of an irradiation point is determined within the range of a tumor, at a centroid or a deepest point of a tumor, and the sampling of irradiation angles is made by random sampling or at a predetermined angle interval; and a neutron beam angle is set to a vector direction from the irradiation point to the centroid or the deepest point of the tumor;
a calculation part (712) for calculating an evaluation value corresponding to each set of irradiation point and irradiation angle, wherein the evaluation value is obtained by sequentially calculating weighting factors of every organ in an organ track and summing up the weighing factors of every organ except the tumor, and the weighing factor of the organ is calculated according to a neutron intensity, a radiation sensitivity factor of the organ and a boron concentration of the organ, and after the evaluation values corresponding to the irradiation points and the irradiation angles are calculated, a quality of each set of irradiation point and the irradiation angle is sorted according to the evaluation values; and
a selection part (713) for selecting one optimal feasible set of irradiation point and irradiation angle from all sampled irradiation points and irradiation angles according to the evaluation values calculated by the calculation part,
**characterised in that**
the selection part (713) removes unfeasible irradiation points and irradiation angles in an actual irradiation process from all the sampled irradiation points and irradiation angles and selects the optimal feasible set of the irradiation point and irradiation angle before the calculation of the evaluation values, and after sorting every set of irradiation point and irradiation angle, the selection part (713) sequentially verifies whether each of the sets of irradiation points and irradiation angles is feasible from the best to the worst until the optimal feasible set of the irradiation point and irradiation angle is found.

2.   The irradiation parameter selection apparatus according to claim 1, wherein the calculation part (712) calculates a depth at which the neutron beam enters a patient and a type of the organ through which the neutron beam passes, and then determines whether the tumor is within a range of the maximum treatable depth corresponding to the set of the irradiation point and the irradiation angle according to track information of the neutron beam passing through a human body, if yes, calculates the evaluation value corresponding to the set of the irradiation point and the irradiation angle according to the track information in combination with data of the boron concentration in the organ, the radiation sensitivity factor of the organ, the characteristic information of the neutron beam and the like, which are set by a user.

3.   A usage method of the irradiation parameter selection apparatus according to any one of claims 1 to 2, the method comprising the following steps:

the sampling part reads an image of a patient, such as CT or MRI or PET-CT that has a clear anatomy of a human body, defines an outline of each organ, tissue and tumor one by one, provides settings of material type and density, and samplings an irradiation point and an irradiation angle of a neutron beam after defining the outline, material and density;
the calculation part calculates a track in the organ through which the neutron beam passes, that is, calculates the type and thickness of the organ that the neutron beam passes through after entering the human body, determines whether the tumor is within the range of the maximum treatable depth after obtaining the track information of the neutron beam passing through the human body, if yes, calculates the evaluation value corresponding to the irradiation point and the irradiation angle according to the track information in combination with data of the boron concentration in the organ, the radiation sensitivity factor of the organ, the characteristic information of the neutron beam and the like, which are set by a user, if not, scores the worst evaluation value, and records the irradiation point, the irradiation angle and the corresponding evaluation value after the calculation of the evaluation value; and

the selection part selects one optimal feasible set of the radiation parameters from all the sampled radiation parameters.

4. The usage method of the irradiation parameter selection apparatus according to claim 3, wherein a neutron beam angle is set to a vector direction from the irradiation point to the centroid or the deepest point of the tumor.

5. The usage method of the irradiation parameter selection apparatus according to claim 3, wherein the calculation part outputs the data of the irradiation points, the irradiation angles and the corresponding evaluation values in a form of 3D or 2D graph.

6. The usage method of the irradiation parameter selection apparatus according to any one of claims 3 to 5, wherein the selection process of the selection part is performed entirely automatically by an associated device or is partially manually performed.

7. A control system for controlling a neutron capture therapy equipment comprising a mounting table for placing a patient, the control system comprising:

the irradiation parameter selection apparatus (71) according to any one of claims 1 to 2;
a conversion part (72) for converting the parameters of the optimal feasible irradiation point and irradiation angle into coordinate parameters that the mounting table needs to be moved in place;
an adjustment part (73) for adjusting the mounting table to a coordinate position obtained from the conversion part (72); and
a start-stop part (74) for controlling start and stop of irradiation of the neutron beam.

8. The control system according to claim 7, wherein the conversion part converts the parameters of the optimal feasible radiation point and radiation angle into the coordinate parameters that the mounting table needs to be moved in place during the irradiation process according to CT/MRI/PET-CT information of the patient, positioning information, structure information of the mounting table and the like.

9. A usage method of the control system according to claim 7 or 8, comprising the following steps:

the irradiation parameter selection apparatus selects the optimal feasible irradiation point and irradiation angle;
the conversion part converts the parameters of the optimal feasible irradiation point and irradiation angle into the coordinate parameters that the mounting table needs to be moved in place;
the adjustment part adjusts the mounting table to the coordinate position obtained from the conversion part.

10. The usage method of the control system according to claim 9, **characterized in that**, the calculation part outputs the data of every set of irradiation point and irradiation angle, and the corresponding evaluation value in a form of 3D or 2D graph.

**Patentansprüche**

1. Eine Bestrahlungsparameter-Auswahleinrichtung (71) für einen Neutronenstrahl, wobei Bestrahlungsparameter Bestrahlungspunkte und Bestrahlungswinkel umfassen, wobei die Bestrahlungsparameter-Auswahleinrichtung (71) umfasst:

ein Abtastungsteil (711) zum Abtasten mehrerer Sätze von Bestrahlungspunkten und Bestrahlungswinkeln, wobei eine Abtastung der Bestrahlungspunkte und der Bestrahlungswinkel eine Vorwärtsabtastung ist, wobei eine Position eines Bestrahlungspunkts außerhalb des menschlichen Körpers bestimmt wird und die Abtastung sequenziell in einem festen Winkelintervall oder einem festen Distanzintervall erfolgt, oder die Abtastung zufällig erfolgt; oder eine Rückwärtsabtastung, wobei eine Position eines Bestrahlungspunkts innerhalb des Bereichs eines Tumors an einem Schwerpunkt oder einem tiefsten Punkt eines Tumors bestimmt wird, und die Abtastung der Bestrahlungswinkel durch zufällige Abtastung oder in einem vorgegebenen Winkelintervall erfolgt; und ein Neutronenstrahlwinkel auf eine Vektorrichtung vom Bestrahlungspunkt zum Schwerpunkt oder zum tiefsten Punkt des Tumors eingestellt wird;

ein Berechnungsteil (712) zum Berechnen eines Bewertungswerts, der jedem Satz von Bestrahlungspunkt und Bestrahlungswinkel entspricht, wobei der Bewertungswert durch sequenzielle Berechnung der Gewichtungsfaktoren jedes Organs in einer Organbahn und durch Addition der Gewichtungsfaktoren jedes Organs außer dem Tumor erhalten wird, und der Gewichtungsfaktor des Organs gemäß einer Neutronenintensität, einem Strahlungsempfindlichkeitsfaktor des Organs und einer Bor-Konzentration des Organs berechnet wird, und nachdem die Bewertungswerte, die den Bestrahlungspunkten und den Bestrahlungswinkeln entsprechen, berechnet wurden, eine Qualität jedes Satzes von Bestrahlungspunkt und Bestrahlungswinkel gemäß den Bewertungswerten sortiert wird; und

ein Auswahlteil (713) zum Auswählen eines optimalen machbaren Satzes von Bestrahlungspunkt und Bestrahlungswinkel aus allen abgetasteten Bestrahlungspunkten und Bestrahlungswinkeln gemäß den Bewertungswerten, die durch den Berechnungsteil berechnet wurden, **dadurch gekennzeichnet, dass** der Auswahlteil (713) unmachbare Bestrahlungspunkte und Bestrahlungswinkel in einem tatsächlichen Bestrahlungsprozess aus allen abgetasteten Bestrahlungspunkten und Bestrahlungswinkeln entfernt und den optimalen machbaren Satz von Bestrahlungspunkt und Bestrahlungswinkel vor der Berechnung der Bewertungswerte auswählt, und nach der Sortierung jedes Satzes von Bestrahlungspunkt und Bestrahlungswinkel der Auswahlteil (713) nacheinander überprüft, ob jeder der Sätze von Bestrahlungspunkten und Bestrahlungswinkeln von der besten bis zur schlechtesten Option machbar ist, bis der optimale machbare Satz von Bestrahlungspunkt und Bestrahlungswinkel gefunden ist.

2. Die Bestrahlungsparameter-Auswahleinrichtung nach Anspruch 1, wobei der Berechnungsteil (712) eine Tiefe berechnet, in der der Neutronenstrahl in einen Patienten eindringt, und eine Art des Organs, durch das der Neutronenstrahl hindurchgeht, und dann bestimmt, ob der Tumor innerhalb eines Bereichs der maximal behandelbaren Tiefe entsprechend dem Satz von Bestrahlungspunkt und Bestrahlungswinkel liegt, gemäß Bahninformationen des Neutronenstrahls, der durch einen menschlichen Körper hindurchgeht, wenn ja, Berechnen des Bewertungswerts entsprechend dem Satz von Bestrahlungspunkt und Bestrahlungswinkel gemäß den Bahninformationen in Kombination mit Daten der Bor-Konzentration im Organ, dem Strahlungsempfindlichkeitsfaktor des Organs, den Charakteristikinformationen des Neutronenstrahls und Ähnlichem, die vom Benutzer festgelegt sind.

3. Ein Verwendungsverfahren der Bestrahlungsparameter-Auswahleinrichtung nach einem der Ansprüche 1 bis 2, umfassend die folgenden Schritte:

das Abtastungsteil liest ein Bild eines Patienten, wie CT oder MRT oder PET-CT, das eine klare Anatomie eines menschlichen Körpers aufweist, definiert eine Umrisslinie jedes Organs, Gewebes und Tumors einzeln, stellt Einstellungen des Materialtyps und der Dichte bereit und tastet einen Bestrahlungspunkt und einen Bestrahlungswinkel eines Neutronenstrahls ab, nachdem der Umriss, das Material und die Dichte definiert wurden;

der Berechnungsteil berechnet eine Bahn in dem Organ, durch das der Neutronenstrahl hindurchgeht, das heißt, berechnet die Art und Dicke des Organs, durch das der Neutronenstrahl hindurchgeht, nachdem er in den menschlichen Körper eingetreten ist, bestimmt, ob der Tumor innerhalb des Bereichs der maximal behandelbaren Tiefe liegt, nachdem die Bahninformationen des Neutronenstrahls, der durch den menschlichen Körper hindurchgeht, erhalten wurden, wenn ja, berechnet den Bewertungswert entsprechend dem Bestrahlungspunkt und dem Bestrahlungswinkel gemäß den Bahninformationen in Kombination mit Daten der Bor-Konzentration im Organ, dem Strahlungsempfindlichkeitsfaktor des Organs, den Charakteristikinformationen des Neutronenstrahls und Ähnlichem, die vom Benutzer festgelegt sind, wenn nein, weist den schlechtesten Bewertungswert zu und zeichnet den Bestrahlungspunkt, den Bestrahlungswinkel und den entsprechenden Bewertungswert nach der Berechnung des Bewertungswerts auf; und

das Auswahlteil wählt einen optimalen machbaren Satz der Bestrahlungsparameter aus allen abgetasteten Bestrahlungsparametern aus.

4. Das Verwendungsverfahren der Bestrahlungsparameter-Auswahleinrichtung nach Anspruch 3, wobei ein Neutronenstrahlwinkel auf eine Vektorrichtung vom Bestrahlungspunkt zum Schwerpunkt oder zum tiefsten Punkt des Tumors eingestellt ist.

5. Das Verwendungsverfahren der Bestrahlungsparameter-Auswahleinrichtung nach Anspruch 3, wobei der Berechnungsteil die Daten der Bestrahlungspunkte, der Bestrahlungswinkel und der entsprechenden Bewertungswerte in Form eines 3D- oder 2D-Diagramms ausgibt.

6. Das Verwendungsverfahren der Bestrahlungsparameter-Auswahleinrichtung nach einem der Ansprüche 3 bis 5, wobei der Auswahlprozess des Auswahlteils vollständig automatisch von einer zugehörigen Vorrichtung durchgeführt wird oder teilweise manuell erfolgt.

7. Ein Steuersystem zum Steuern eines Neutroneneinfangtherapiegeräts, umfassend einen Montagetisch zum Platzieren eines Patienten, wobei das Steuersystem umfasst:

   die Bestrahlungsparameter-Auswahleinrichtung (71) nach einem der Ansprüche 1 bis 2;
   ein Konversionsteil (72) zum Konvertieren der Parameter des optimalen machbaren Bestrahlungspunkts und des Bestrahlungswinkels in Koordinatenparameter, die der Montagetisch zur Positionierung benötigt;
   ein Anpassungsteil (73) zum Anpassen des Montagetisches an die vom Konversionsteil (72) erhaltene Koordinatenposition; und
   ein Start-Stopp-Teil (74) zum Steuern des Startens und Stoppen der Bestrahlung des Neutronenstrahls.

8. Das Steuersystem nach Anspruch 7, wobei das Konversionsteil die Parameter des optimalen machbaren Bestrahlungspunkts und Bestrahlungswinkels in die Koordinatenparameter konvertiert, die der Montagetisch während des Bestrahlungsprozesses zur Positionierung benötigt, gemäß CT/MRT/PET-CT-Informationen des Patienten, Positionierungsinformationen, Strukturinformationen des Montagetisches und Ähnlichem.

9. Ein Verwendungsverfahren des Steuersystems nach Anspruch 7 oder 8, umfassend die folgenden Schritte:

   die Bestrahlungsparameter-Auswahleinrichtung wählt den optimalen machbaren Bestrahlungspunkt und Bestrahlungswinkel aus;
   das Konversionsteil konvertiert die Parameter des optimalen machbaren Bestrahlungspunkts und Bestrahlungswinkels in die Koordinatenparameter, die der Montagetisch zur Positionierung benötigt;
   das Anpassungsteil stellt den Montagetisch auf die vom Konversionsteil erhaltene Koordinatenposition ein.

10. Das Verwendungsverfahren des Steuersystems nach Anspruch 9, **dadurch gekennzeichnet, dass** der Berechnungsteil die Daten jedes Satzes von Bestrahlungspunkt und Bestrahlungswinkel und den entsprechenden Bewertungswert in Form eines 3D- oder 2D-Diagramms ausgibt.

## Revendications

1. Appareil de sélection de paramètres d'irradiation (71) pour un faisceau de neutrons, dans lequel les paramètres d'irradiation comprennent les points d'irradiation et les angles d'irradiation, l'appareil de sélection des paramètres d'irradiation (71) comprenant:

   une partie d'échantillonnage (711) destinée à échantillonner plusieurs ensembles de points d'irradiation et d'angles d'irradiation, dans lequel l'échantillonnage des points d'irradiation et des angles d'irradiation étant un échantillonnage direct, dans lequel la position d'un point d'irradiation est déterminée à l'extérieur du corps humain et l'échantillonnage est effectué de manière séquentielle à un intervalle d'angle fixe ou à un intervalle de distance fixe, ou l'échantillonnage est effectué de manière aléatoire; ou un échantillonnage inverse, dans lequel la position d'un point d'irradiation est déterminée dans la plage de volume d'une tumeur, à un centroïde ou à un point le plus profond d'une tumeur, et l'échantillonnage des angles d'irradiation est effectué par échantillonnage aléatoire ou à un intervalle d'angle prédéterminé; et un angle de faisceau de neutrons est fixé à une direction vectorielle du point d'irradiation au centroïde ou au point le plus profond de la tumeur;
   une partie de calcul (712) destinée à calculer une valeur d'évaluation correspondant à chaque ensemble de point d'irradiation et d'angle d'irradiation, dans lequel la valeur d'évaluation est obtenue par calcul séquentiel de facteurs de pondération de chaque organe dans une trajectoire d'organe et par addition des facteurs de pondération de chaque organe à l'exception de la tumeur, et le facteur de pondération de l'organe est calculé en fonction d'une intensité de neutrons, d'un facteur de sensibilité aux rayonnements de l'organe et d'une concentration de bore de l'organe, et après le calcul des valeurs d'évaluation correspondant aux points d'irradiation et aux angles d'irradiation, la qualité de chaque ensemble de points d'irradiation et d'angles d'irradiation est triée en fonction des valeurs d'évaluation; et
   une partie de sélection (713) destinée à sélectionner un ensemble optimal réalisable de points d'irradiation et d'angles d'irradiation parmi tous les points d'irradiation et angles d'irradiation échantillonnés en fonction des valeurs d'évaluation calculées par la partie de calcul, **caractérisé en ce que** la partie de sélection (713) supprime les points d'irradiation et les angles d'irradiation irréalisables dans un processus d'irradiation réel parmi tous les points d'irradiation et angles d'irradiation échantillonnés et sélectionne l'ensemble optimal réalisable du point d'irradiation et de l'angle d'irradiation avant le calcul des valeurs d'évaluation, et après avoir trié chaque ensemble de point d'irradiation et d'angle d'irradiation, la partie sélection (713) vérifie séquentiellement si chacun des ensembles de points d'irradiation et d'angles d'irradiation est réalisable, du meilleur au plus mauvais, jusqu'à ce que l'ensemble optimal réalisable de point d'irradiation et d'angle d'irradiation soit trouvé.

2. Appareil de sélection de paramètres d'irradiation (71) selon la revendication 1, dans lequel la partie calcul (712) calcule une profondeur à laquelle le faisceau de neutrons pénètre dans un patient et un type d'organe à travers lequel passe le faisceau de neutrons, puis détermine si la tumeur se trouve dans une plage de profondeur maximale traitable correspondant à l'ensemble du point d'irradiation et de l'angle d'irradiation en fonction des informations de trajectoire du faisceau de neutrons traversant un corps humain, dans l'affirmative, calcule la valeur d'évaluation correspondant à l'ensemble du point d'irradiation et de l'angle d'irradiation selon les informations de trajectoire en combinaison avec les données de concentration de bore dans l'organe, le facteur de sensibilité aux rayonnements de l'organe, les informations caractéristiques du faisceau de neutrons et autres, qui sont définis par un utilisateur.

3. Procédé d'utilisation de l'appareil de sélection de paramètres d'irradiation selon l'une quelconque des revendications 1 à 2, le procédé comprenant les étapes suivantes:

la partie échantillonnage lit une image d'un patient, telle que la tomographie par ordinateur ou IRM ou PET-CT, qui présente une anatomie claire du corps humain, définit un contour de chaque organe, tissu et tumeur un par un, fournit des paramètres de type de matière et de densité, et échantillonne un point d'irradiation et un angle d'irradiation d'un faisceau de neutrons après avoir défini le contour, la matière et la densité;
la partie de calcul calcule une trajectoire dans l'organe traversé par le faisceau de neutrons, c'est-à-dire qu'elle calcule le type et l'épaisseur de l'organe traversé par le faisceau de neutrons après avoir pénétré dans le corps humain, détermine si la tumeur se situe dans la plage de la profondeur maximale traitable après avoir obtenu les informations de trajectoire du faisceau de neutrons traversant le corps humain, dans l'affirmative, calcule la valeur d'évaluation correspondant au point d'irradiation et à l'angle d'irradiation selon les informations de trajectoire en combinaison avec les données de concentration de bore dans l'organe, le facteur de sensibilité aux rayonnements de l'organe, les informations caractéristiques du faisceau de neutrons et autres, qui sont définis par un utilisateur, dans le cas contraire, note la valeur d'évaluation la plus mauvaise, et enregistre le point d'irradiation, l'angle d'irradiation et la valeur d'évaluation correspondante après le calcul de la valeur d'évaluation; et
la partie de sélection sélectionne un ensemble optimal réalisable des paramètres d'irradiation parmi tous les paramètres d'irradiation échantillonnés.

4. Procédé d'utilisation de l'appareil de sélection de paramètres d'irradiation selon la revendication 3, dans lequel un angle du faisceau de neutrons est fixé à une direction vectorielle allant du point d'irradiation au centroïde ou au point le plus profond de la tumeur.

5. Procédé d'utilisation de l'appareil de sélection de paramètres d'irradiation selon la revendication 3, dans lequel la partie de calcul produit les données des points d'irradiation, des angles d'irradiation et des valeurs d'évaluation correspondantes sous la forme d'un graphique 3D ou 2D.

6. Procédé d'utilisation de l'appareil de sélection de paramètres d'irradiation selon l'une des revendications 3 à 5, dans lequel le processus de sélection de la partie de sélection est exécuté entièrement automatiquement par un dispositif associé ou est partiellement exécuté manuellement.

7. Système de commande permettant de commander d'un équipement de thérapie par capture de neutrons comprenant une table support pour placer un patient, le système de commande comprenant:

l'appareil de sélection de paramètres d'irradiation (71) selon l'une quelconque des revendications 1 à 2;
une partie de conversion (72) destinée à convertir les paramètres du point d'irradiation optimal réalisable et de l'angle d'irradiation en paramètres de coordonnées dont la table support a besoin pour être déplacée;
une partie de réglage (73) destinée à ajuster la table support dans une position de coordonnées obtenue à partir de la partie de conversion (72); et
une partie marche-arrêt (74) destinée à commander le démarrage et l'arrêt de l'irradiation du faisceau de neutrons.

8. Système de commande selon la revendication 7, dans lequel la partie conversion convertit les paramètres du point d'irradiation optimal réalisable et de l'angle d'irradiation en paramètres de coordonnées dont la table supporta besoin pour être déplacée pendant le processus d'irradiation en fonction des informations CT/MRI/PET-CT du patient, des informations de positionnement, des informations sur la structure de la table support et autres.

9. Procédé d'utilisation du système de commande selon la revendication 7 ou 8, comprenant les étapes suivantes, dans lequel:

l'appareil de sélection des paramètres d'irradiation sélectionne le point d'irradiation et l'angle d'irradiation optimaux réalisables;
la partie de conversion convertit les paramètres du point d'irradiation optimal réalisable et de l'angle d'irradiation en paramètres de coordonnées dont la table de montage a besoin pour être déplacée;
la partie de réglage ajuste la table support dans la position de coordonnées obtenue par la partie conversion.

10. Procédé d'utilisation du système de commande selon la revendication 9, **caractérisée en ce que** la partie de calcul fournit les données de chaque ensemble de point d'irradiation et d'angle d'irradiation, ainsi que la valeur d'évaluation correspondante sous la forme d'un graphique en 3D ou en 2D.

**FIG. 1**

$$^{1}\text{n} + {}^{10}\text{B} \longrightarrow {}^{11}\text{B*}$$

$$\longrightarrow {}^{7}\text{Li} + {}^{4}\text{He} + 2.79 \text{ MeV} \qquad (6.1\%)$$

$$\longrightarrow {}^{7}\text{Li*} + {}^{4}\text{He} + 2.31 \text{ MeV} \qquad (93.9\%)$$

$$\downarrow$$

$$^{7}\text{Li} + \gamma\text{-ray} (0.48 \text{ MeV})$$

**FIG. 2**

**FIG. 3**

711 — Sampling Part

712 — Calculation Part

71

713 — Selection Part

Conversion Part — 72

Adjustment Part — 73

Star/stop Part — 74

**FIG. 4**

Reading medical
image data

Defining or reading
outlines of an organ,
a tissue and a tumor

Defining materials'
types and densities
of the organ, tissue
and tumor

Sampling an
irradiation angle of
the beam

Calculating the
beam track through
the organ

Reading the
boron
concentration
in the organ

Reading the
radiation
sensitive
factor

Reading the
beam
characteristic
information

Whether
is the tumor fully
located within the effective
treatment depth

No → Scoring
the worst
evaluation
factor

Yes

Calculating the
evaluation factor

Recording the
irradiation
conditions and the
calculation results

**FIG. 5**

Incidence of
neutron
beam

Skin    Skull    Brain tissue    Tumor    Brain tissue    Skull    Skin

**FIG. 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018277278 A1, LIU YUAN-HAO
- CN 105457172 A
- WO 201510790248 A